# EUROPEAN PATENT SPECIFICATION

(11) **EP 1 540 612 B1**
(45) Date of publication and mention of the grant of the patent: **02.12.2009**
(21) Application number: 03749112.3
(22) Date of filing: 22.08.2003
(51) Int. Cl.: E04H 4/16, B08B 7/04, G01N 27/416, E04H 3/16

(54) **POOL CLEANER WITH ON-BOARD WATER ANALYSIS, DATA RECORDING AND TRANSMISSION DEVICE**
SWIMMING-POOL-REINIGUNGSVORRICHTUNG MIT EINRICHTUNG ZUR ONBOARD-WASSERANALYSE, DATENAUFZEICHNUNG UND DATENÜBERTRAGUNG
SYSTEME DE NETTOYAGE DE PISCINE EQUIPE D'UN DISPOSITIF EMBARQUE POUR EFFECTUER DES ANALYSES D'EAU AINSI QUE POUR ENREGISTRER ET TRANSMETTRE DES DONNEES

(30) Priority: 23.08.2002 US 405507 P
(43) Date of publication of application: 15.06.2005
(73) Proprietor: Aqua Products Inc., Cedar Grove, NJ 07009 (US)
(72) Inventor: PORAT, Joseph, Del Ray Beach, FL 33483 (US)
(74) Representative: Gervasi, Gemma
(86) International application number: PCT/US2003/026352
(87) International publication number: WO 2004/019295

(56) References cited:
- DE-A1- 4 221 086
- US-A- 5 218 304
- US-A- 5 337 434
- US-A- 5 507 058
- US-A1- 2001 050 093
- US-B1- 6 222 449

## Description

### Field of the Invention

This invention relates to sensors and data recording and display devices used to analyze various characteristics of swimming pool water for the purpose of determining the need to add additional chemicals to change or to adjust, for example, the chlorine content and pH.

### Background of the Invention

Various automated sensing and analysis devices have been developed to measure, record and display the level of free chlorine, oxidation reduction potential ("redox"), the pH value, hardness, total dissolved solids ("TDS") temperature and other characteristics and conditions of swimming pool water in order to simplify and facilitate the maintenance of the conditions within a desired or optimum range. These devices include a sensor capable of periodically sampling and measuring the pH and the free chlorine ion level of water. The results of the analysis are converted to digital form by circuitry known to the art and stored in the memory of a microprocessor. The stored data can be accessed for display to determine whether any maintenance action is required to restore the conditions of the water to the desired pH and chlorine content range. These devices can also be activated to obtain a display of conditions on demand. The display is via an LCD screen of the type commonly found on pagers and cellular telephones.

The water is typically sampled by means installed in the piping associated with the water filter treatment apparatus. The purchase price, installation expense and maintenance costs associated with this prior art system can be substantial. Means for accessing a display or read-out must also be provided. Thus, the prior art requires that a complete, separate system and apparatus be installed and periodically checked at a location that may not be particularly convenient, such as a filter room or filter house located under the pool or in an even more remote location.

In addition to being inconvenient, the systems of the prior art require the owner to remain vigilant to weather conditions, such as outdoor temperatures, extent of sunshine and the like, which can have a marked effect on the frequency with which conditions of the pool water can change. Quite simply, the owner must remember to check the system in order to maintain the water in a safe and healthy condition.

For most residential pools, the home owner and/or his family members, a gardener or other employee with other principal duties will be responsible for pool maintenance. Since water conditions can fall outside of the desired or safe range without a visible manifestation, an improved system is required to alert the person responsible for monitoring pool maintenance that conditions should be checked and some remedial action be taken.

It is therefore an object of the present invention to provide sensing, analysis, recording, data processing and display apparatus that is integrated directly into a robotic pool cleaner assembly and one or more ancillary components, and which is readily accessible either on a component of the assembly or on the remote stationary or hand-held device dedicated to this purpose.

A further object of the invention is to provide an improved apparatus and method for monitoring and analyzing the condition of the water in a swimming pool or other tank or vessel by directly reading the conditions in the volume of liquid contained in the pool rather than liquid passing through a conduit or pipe and for providing a display of the relevant data reflecting such conditions at predetermined periodic intervals and/or on demand.

It is yet a further object of the invention to provide means and a method for displaying such data that will not require modifications of the filter or piping system used to treat and carry the water, to thereby minimize the expenses associated with obtaining and displaying such information.

It is yet another object of the invention to provide an automatic apparatus and method that will provide a signal alerting a change in conditions requiring maintenance or remedial action and to also provide customized instructions and data as to the maintenance or remedial action required, including, for example, the amount and type of one or more chemicals that must be added to restore the condition of the water to a predetermined desired level or range based upon the specific type of chemicals that have been used historically in the maintenance of the pool being monitored.

### Summary of the Invention

The above objects and other advantages are achieved by the invention in which a robotic pool cleaner used to clean the bottom wall of a pool or tank is provided with on-board sensor and analysis means for measuring one or more characteristics or conditions of the water in the pool, a programmed microprocessor for receiving data from the sensor and analysis means, data storage means associated with the microprocessor, data transmitting means and associated display means for displaying data derived from the sensor means.

As used herein, the term "sensor" means any one or more of commercially available devices that have the ability to sample water in which the device is immersed, analyze the content of the water for one or more parameters including, for example the level of free chlorine, oxidation reduction potential ("redox"), the pH value, hardness, total dissolved solids ("TDS") and temperature, and to store or transmit for storage the results of the analysis in digital form.

Sensors suitable for use in the practice of the invention are available from commercial sources including the following : Omega USA of Stamford, CT. , www.omega.com; Honeywell Sensing and Control of Freeport, IL., http//content.honeywell.com/sensing/products/analytical; Signet Scientific Company, http://www.gfsignet.com; Hanna Instruments S.P.A. of Padova, Italy, http://www.hannainst.com; and ZD Instrument Co. of Jiangsu Province, China; http://www.zdinstrument.com.

When a remote power supply is used, the data is transmitted from the operating submerged pool cleaner via a separate data cable that is attached to the power cable or contained within the same insulating cover as the power conductors. The data transmission line(s) can be a shielded coaxial cable. The data line is then attached to the data display device. The connection can be permanent or via a jack when a separate data line is employed.

In one preferred embodiment, where the robotic pool cleaner receives its power from a remote power supply located outside of the pool, the data display means is associated with the power supply, either in a separate housing mounted on the exterior of the power supply or by incorporating the display means into the power supply housing. In a particularly preferred embodiment, the display means is interactive and includes a switch for activating the display screen and, in an especially preferred embodiment, a keypad or function keys that is operable by the user to advance the various characteristics and conditions that have been stored in the microprocessor's memory device and to selectively view the instructions for remedial action, such as chemical type and quantity that is to be added to restore the desired condition of the pool water.

In a second preferred embodiment, the pool cleaner is powered by an integral rechargeable battery and is provided with an antenna, the transmitting end of which floats on the surface of the pool water to transmit a signal to either or both of a stationary or a hand-held device. The transmission of data can be programmed for a periodic transmission or in response to signal from the receiver/display device that is passed through the floating antenna to the microprocessor which is activated to transmit stored data from the last test or series of tests. In a particularly preferred embodiment, the hand-held display device can be configured similarly to a cell phone with a case, antenna, display screen, keypad and battery power supply. Alternatively, the hand-held device can be configured similar to a television remote control device.

The sensor means can be positioned on the interior of the housing of the pool cleaner in an active flow path of water being drawn into the pool cleaner filter assembly. This location provides an accurate sampling of the actual conditions in the pool and also protects the sensors from damage.

Alternatively, the sensors can be mounted on the exterior of the housing in a protective casing through which casing pool water to be sampled will flow. Sensor conductor leads pass through openings in the cleaner housing to the microprocessor memory unit.

The power requirements for the sensor, microprocessor and transmitter are obtained from the power supplied to the pool cleaner to operate the pump or pumps and any drive motor or motors. As noted above, this can be a remote power source located outside of the pool or a rechargeable battery that is also positioned inside the pool cleaner housing.

For a battery-powered pool cleaner, a separate antenna wire must be provided, with the free end floating at the surface of the pool water. The antenna is preferably attached via a plug and jack assembly passing through the pool cleaner housing that is attached to the microprocessor and transmitter circuit. In this way, the antenna can be removed and coiled for storage when the cleaner is removed from the pool for maintenance or storage. Alternatively, the antenna can be attached to a manually operated reel or drawn from an automatic coiling device located on the exterior or interior of the pool cleaner housing.

As previously noted, the on-board sensor means can include means for analyzing any one or more of the water characteristics and conditions that are presently available or that may be developed in the future. A separate power source can be provided to the sensor unit(s) to permit their operation even when the pool cleaner is not operating to clean the pool. The separate power source can be a circuit supplied by the remote power supply or a separate rechargeable or dry cell battery. In this embodiment, the pool cleaner is left in the pool and will display or even signal a change in conditions requiring remedial action to restore the pool water.

In the case of either the remote power supply or the integral battery power supply, the apparatus can include a remote hand-held display device having the characteristics of a pager, or a more sophisticated device that has additional interactive functional capabilities than the pager that can be carried by the user to interrogate the stored data.

The programmed microprocessor can include the generation of an audible and/or visible warning signal to indicate that one or more of the measured conditions is outside of a predetermined acceptable range. For example, in heated swimming pools, the program for a temperature sensor can include an audible alarm to indicate when the water temperature has dropped below or exceeded a predetermined value. Such a condition might arise in the event of a failure of the pool's heating element and/or its associated temperature sensor and thermostatic control device. A rise in water temperature will also cause more chlorine to be released into the atmosphere and will accelerate the replacement requirement for this chemical. Prompt remedial action can save time and money in restoring the desired balance.

The most common maintenance requirement for swimming pools is the addition of a source of chlorine ions in order to inhibit the growth of bacteria, algae and the like. The effect of direct sunlight and an increase in temperature is well known to reduce the level of chlorine ion. Thus, in a preferred embodiment of the invention, a chlorine sensor is included and the microprocessor is programmed to provide a signal to indicate when the chlorine level drops below the desired predetermined level.

In an especially preferred embodiment, the microprocessor and memory device is programmed to permit the owner to identify the particular type and form of chlorine-containing chemical that has historically been used in the treatment of the pool. For example, HTH is available in the form of a liquid additive, crystals and powder. A microprocessor is programmed to display a recommended amount of the particular type and form of chlorine ion chemical that must be added based upon the entry of data that includes the volume of water in the pool that must be treated. The program can also include other parameters, such as water temperature and sun load in identifying the volume and/or weight of chemical product to be added.

In one particularly preferred embodiment, the microprocessor is programmed with information about all of the known chemical products commonly used in a particular geographical area where the pool cleaner is to be sold. The programmed information can include trademarks and brand names. Algorithms are provided to account for water temperature, sun loads, water hardness ranges, pH ranges and such other parameters are customarily measured.

The user is provided with one or more menus from which are selected the particular brand and/or type of chemical used to maintain the pool in which the pool cleaner of the invention is to be used. The volume of the water in the pool is also entered. Thereafter, during use, the display will include a screen with information as to how much of the previously specific chemical is to be added to restore water conditions within the desired or prescribed range.

As will be apparent to one of ordinary skill in the art, that data from the on-board sensors can also be transmitted to a stationary data display device, such as a personal computer, portable notebook or laptop computer for access by the user. For convenience, all such computers will be referred to as "stationary devices" to distinguish them from the hand-held devices described above. The stationary display device can be located in the user's home. The pool monitoring system can also be incorporated into an advanced household monitoring system of the type that are known to the prior art.

Any of the various types of transmitting methods and apparatus known to the art or developed in the future can be adopted. In the case of a remote power supply, the data display device can be hard-wired to one of more secondary display devices in the user's home. In the case of a battery-powered pool cleaner, one or more stationary receiving antennas can be positioned in the vicinity of the pool to pick-up the data signals from the microprocessor. If necessary or desired, the receiving antennas can be provided with booster to enhance the signal for retransmission.

The data transmission and receiving system of the invention further comprehends the use of the Internet for providing access to the data from other remote locations, either accessible to the owner or to a commercial pool servicing organization. This type of system can also be used by institutions, such as universities, municipal parks departments and the like, to monitor the conditions of numerous pools under their jurisdiction for the purpose of dispatching an employee to correct conditions that are not within acceptable ranges. Use of the invention in such circumstances would permit maintenance personnel to take action promptly on an as-needed basis to enhance efficiency, reduce costs and maximize operations within predetermined safety limits for the benefit of swimmers and others using the pools.

### Brief Description of the Drawings

The invention will be described in more detail below and with reference to the attached drawings in which:
FIG. 1 is a schematic illustration of one preferred embodiment of a pool cleaner of the invention provided with an external sensor array in operation with a remote power supply equipped with a data display screen and interactive display switches;
FIG. 2 is an enlarged front, top, right side perspective view of the power supply of FIG. 1;
FIG. 3 is a schematic illustration of the invention similar to FIG. 1 showing the sensor array positioned on the interior of the pool cleaner housing and its relation to a remote hard-wired data display device and pager and to an alternative hand-held device that communicates interactively with the hard-wired device;
FIG. 4 is a schematic illustration of the invention in which the power is supplied by a battery that is integral with the pool cleaner; and
FIG. 5 is a schematic block diagram of an interactive communications system in accordance with another preferred embodiment which integrates a battery-powered pool cleaner with a plurality of local and remote interactive data display devices and antennas, and an Internet link.

### Detailed Description of the Preferred Embodiments

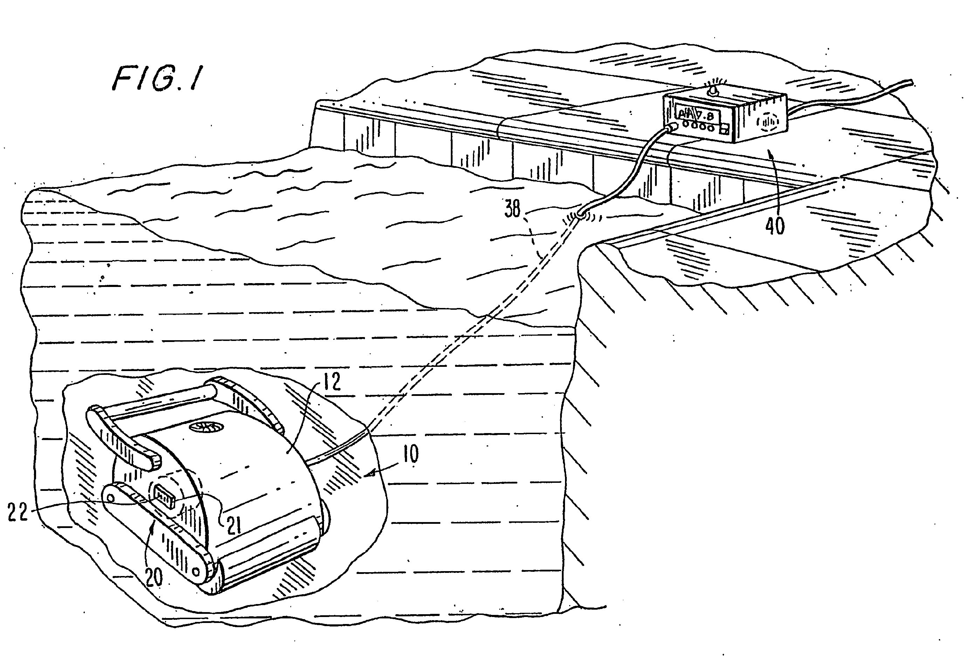

In one preferred embodiment of the invention illustrated in Fig. 1, a pool cleaner generally referred to as 10, is provided with an external sensor array 20 comprising one or more sensors (or sensors/analyzers) 23 contained in a protective housing 21 provided with one or more water sampling inlets 22. Each sensor 23 is adapted to sense a respective condition of the water in the pool and is therefore physically disposed in or on the pool cleaner 10 so as to be in sensing relation to the water. In this embodiment, an external power supply 40 is connected to the pool cleaner 10 by power cable 50.

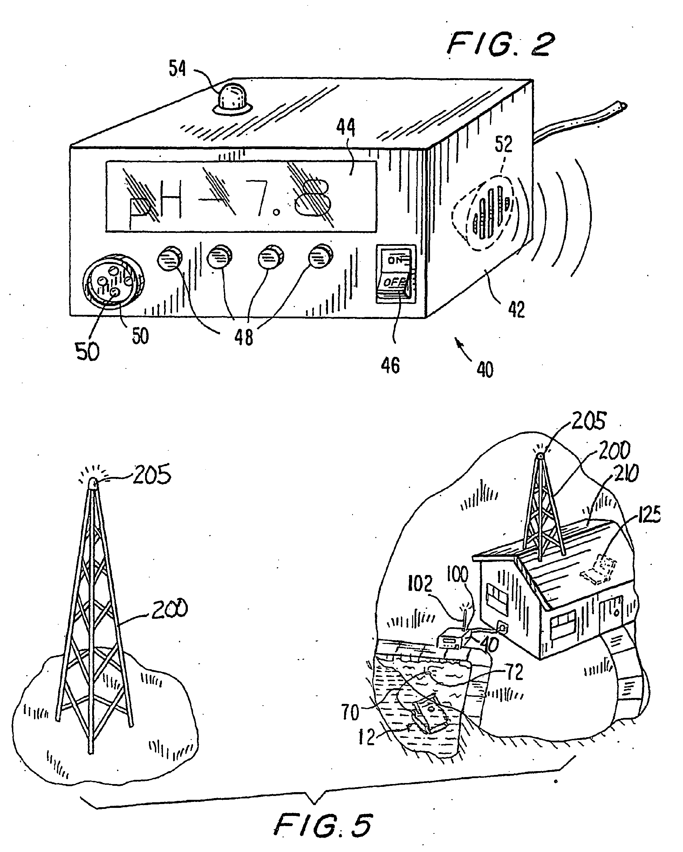

A preferred arrangement of the display and controls as integrated into the casing of the power supply housing 42 is illustrated in Fig. 2. Liquid crystal display 44 is prominently positioned for easy viewing on a front or top side surface of housing 42. A multi-conductor power supply cable outlet 50 is mounted through the housing 42 and includes terminals 51 for receiving information (data) transmitted from the one or more sensors 23 in the pool cleaner 10. The power supply 40 is also provided with an on/off power switch 46 and one or more, but preferably a plurality of, function and characteristic display switches in the form of pushbuttons 48. These function actuators 48 interact with the microprocessor (not illustrated) that is either located in the power supply housing 42 or more preferably, on-board the pool cleaner.

In one preferred method of practicing the invention, the one or more sensors 23 are periodically activated and the data generated processed to determine whether it is within the predetermined acceptable range. In the event that the data is outside the desired range, an audible alarm can be generated from signal unit 52 and/or a visible signal device such as flashing light 54 is activated. Either or both of these signal devices can be omitted or deactivated and the user can interrogate the device to obtain a on-demand display of the data for any one or more of the characteristics stored in the microprocessor's memory device. In a particularly preferred embodiment, the microprocessor is programmed to display the acceptable value or range along with the most recent actual analysis result.

The display also preferably prompts the user with information about the proactive or remedial action to be taken, such as "add chlorine." In this embodiment the microprocessor memory device is programmed with information concerning the specific type (i.e., liquid, crystal, powder) and strength or concentration and brand name of the chemicals historically used to treat the pool to maintain its balance. In this embodiment, the user can be prompted to display the required type and amount of chemical additive to be used, taking into account other variables such as the actual volume of the water contained in the pool, water temperature and the like. The programming of microprocessors with this information for use in an interactive manner is known to the art and/or can be extended in accordance with the teachings of the invention by one of ordinary skill.

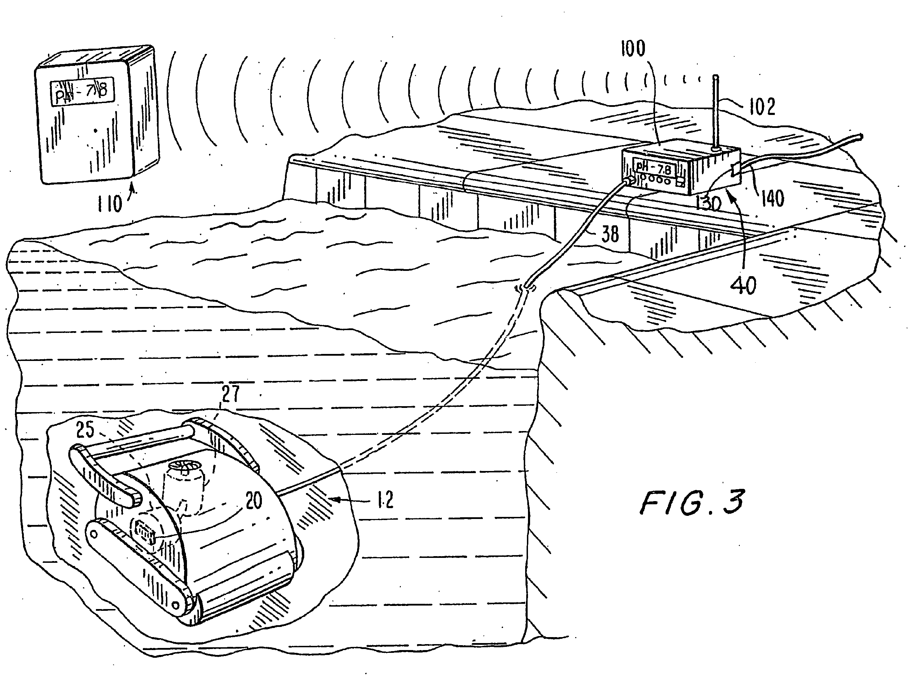

In the alternative embodiment illustrated in Figure 3, showing the interior of a pool cleaner 30 that has been turned upside down for clarity, the sensor array 20 can be incorporated into the casing 25 positioned on the interior of the housing in the active flow path of the water that has been drawn through the filter bag 27. This particular configuration, with the sensors 23 located on the interior of the housing 12, is preferred in order to minimize the possibility of damage by mishandling the pool cleaner 30. This is also a preferred location for the sensor array 20 if the cleaner 30 is to be left in the pool when it is not performing its cleaning operations.

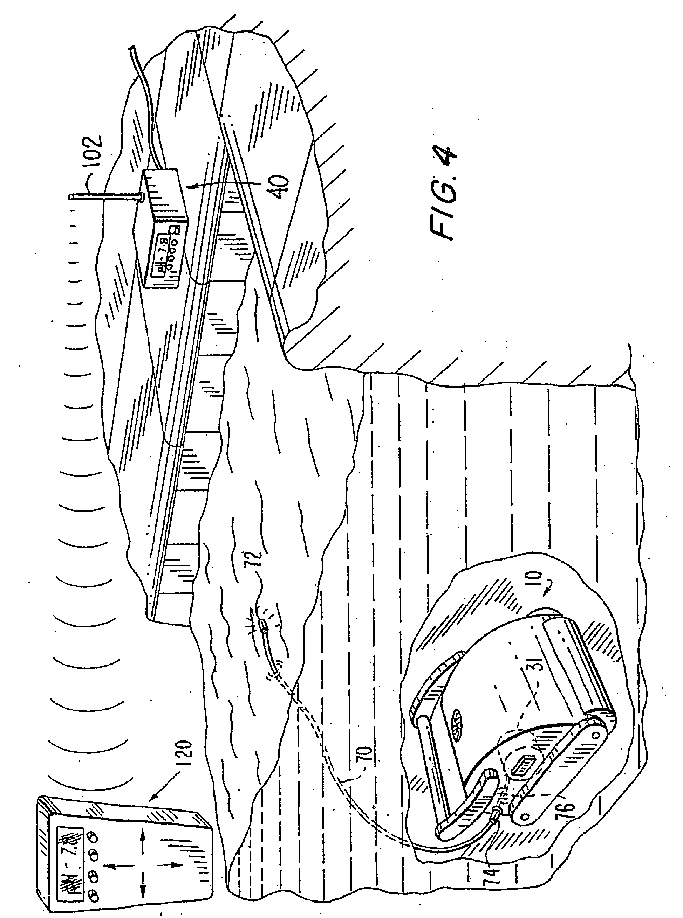

A further embodiment of the invention is illustrated in Fig. 4 where the pool cleaner power is supplied by an on-board rechargeable battery 31, which eliminates the need for an external power cable. In this embodiment, the data must be transmitted through an antenna 70, the transmitting end 72 of which floats on the surface of the pool water.

The antenna 70 can be terminated by a jack and mating plug assembly 74 to permit removal from the housing during storage. A signal transmitting cable 76 links the antenna 70 to the sensor array assembly.

In this embodiment, a remote display device, which can be a hand held display 120 (see Fig. 4), a PC or laptop computer 125 (see Fig. 5) with an appropriate receiving and transmitting antenna 205 of its own or a stationary receiver 100 configured similar to that of the power supply display module 40 described above, can be employed. The stationary receiving transmitter 100 may receive the signal from the floating antenna 70 and retransmit it via antenna 102 to the pager 110 or a more interactive hand-held remote control device 120 (see Fig. 5). Here a separate power cable 38 may be used.

The remote receiving antenna 205 can be installed on a mast 200 dedicated to that use, on a building 210 or home proximate the pool, or at any other convenient location that is within range of the floating antenna.

In the embodiment depicted in Fig. 5, the stationary receiving transmitter 100 receives the signal from the floating antenna 70 and retransmits it via antenna 102 to pager 110 or the more interactive hand-held remote control device 120.

The transmitter/receiver 100 can also be provided with a modem 130 allowing direct access and communication of data via telephone lines 140 or satellite through the Internet to either type of device 110 or 120.

Transmitter receiver 100 can also be integrated into commercially available electronic home monitoring and control systems. The receiver 100 can be hard-wired or connected via antenna 210 and a separate receiver transmitter unit. Such systems typically include signal-type alarms to alert the home owner to conditions requiring attention. The systems also permit the interactive scanning of data that is collected and stored in the system's memory. In a particularly preferred embodiment of the invention, the pool cleaner chemicals are stored in reservoirs connected to automated measuring and injection valves to permit maintenance of the system from the keypad of a PC or other interactive controller keypad forming part of the system.

While the disclosed method and apparatus have been particularly shown and described with respect to the preferred embodiments, it will be apparent to those skilled in the art from the description provided that various modifications in form and detail may be made therein without departing from the scope of the claims. Accordingly, modifications such as those suggested above, but not limited thereto are to be considered within the scope of the invention, which is to be determined by reference to the appended claims.

## Claims

1. A self-propelled robotic pool cleaner for cleaning at least a submerged lower wall of a pool or tank containing water, said pool cleaner comprising:
a cleaner housing;
a sensor array including at least one sensor, said array being supported by said cleaner housing in a position at which each of said at least one sensor is in sensing relation to the water as said pool cleaner moves, each sensor of said array further sensing a specific condition of the water that is related to the proper maintenance and operation of the pool or tank, a power source electronically connected to said sensor array to operate each of said at least one sensor; and
means for outputting information to a remote display on each condition sensed by said at least one sensor on said array as said pool cleaner moves through the water, whereby the specific water condition is sensed at a plurality of locations in the pool or tank.

2. The pool cleaner of claim 1, wherein said sensor array is positioned in the interior of said cleaner housing in an active flow path of water through said pool cleaner.

3. The pool cleaner of claim 1, further comprising a protective housing including at least one water inlet mounted on an exterior surface of said cleaner housing, said sensor array being mounted within said protective housing such that water entering said protective housing through said at least one water inlet flows in contact with said at least one sensor in said sensing relation.

4. The pool cleaner of claim 1, wherein said means for outputting information includes a data cable extending from said cleaner housing for connection to the remote display.

5. The pool cleaner of claim 4, wherein said power source includes a power cable extending from said cleaner housing for connection to a remote source of electrical power, and said data cable comprises at least one conductor within said power cable.

6. The pool cleaner of claim 1, wherein said means for outputting information includes a wireless transmitter for transmitting the information as a wireless signal from said pool cleaner to the remote display.

7. The pool cleaner of claim 1, wherein said wireless transmitter includes an antenna having a free end adapted to float on an upper surface of the water in the pool.

8. The pool cleaner of claim 6, wherein said power supply includes a power cable extending from said cleaner housing for connection to a remote power source.

9. The pool cleaner of claim 1, wherein said power source includes a battery within said cleaner housing.

10. The pool cleaner of claim 1, further comprising a processor for processing data from said sensor array to generate the information to be output.

11. A self-propelled robotic pool cleaning system for cleaning at least a submerged lower wall of a pool or tank containing water, said pool cleaning system comprising:
**a** pool cleaner that includes:
a cleaner housing, and
a sensor array including at least one sensor, each of said at least one sensor being supported by said cleaner housing in a position in sensing relation to the water in that portio of the pool or tank as said pool cleaner moves, each sensor of said at least one sensor sensing a specific condition of the water;
a power source for supplying power to said sensor array to operate each said at least one sensor;
information output means for transmitting information on a condition sensed by each of said at least one sensor; and
a remote display device in communication with said information output means, whereby the water condition is sensed at a plurality of locations in the pool or tank.

12. The system of claim 11, wherein said sensor array is positioned in the interior of said cleaner housing in an active flow path of water.

13. The system of claim 11, said pool cleaner further comprising a protective housing including at least one water inlet mounted on an exterior surface of said cleaner housing, said sensor array being mounted in said protective housing such that water entering said protective housing through said at least one water inlet flows in sensing contact with said at least one sensor.

14. The system of claim 11, wherein said information output means includes a data cable operatively expending from said cleaner housing for connection to said remote display device.

15. The system of claim 14, wherein said power source includes a power cable extending from said cleaner housing for connection to a remote power source, and said data cable comprises at least one conductor in said power cable.

16. The system of claim 15, wherein the power source is positioned in a power supply housing and said remote display device is included in the power supply housing.

17. The system of claim 11, wherein said means for outputting information includes a wireless transmitter for communicating a signal from said pool cleaner to said remote display device.

18. The system of claim 17, wherein said wireless transmitter includes an antenna having a free end adapted to float on the surface of the water.

19. The system of claim 17, wherein said power source includes a rechargeable battery mounted in said cleaner housing.

20. The system of claim 11, wherein said power source includes a power cable extending from said cleaner housing for connection to a remote power source.

21. The system of claim 20, wherein the power source is positioned in a power supply housing and said remote display device is associated with the power supply housing.

22. The system of claim 11, further comprising means for generating an audible and/or visible alarm signal in response to said sensor array detecting a predetermined unacceptable value for a water condition.

23. The system of claim 22, wherein said remote display device includes a display housing and said alarm generating means is associated with said display housing.

24. The system of claim 23, wherein said display device displays an acceptable range for the value of a water condition and a most recent value of the same water condition as determined by a sensor on the pool cleaner.

25. The system of claim 22, wherein said pool cleaner further comprises a processor for processing data from said sensor array to generate the information to be output.

26. The system of claim 25, wherein said processor includes an algorithm employing a predetermined range of values corresponding to each of one or more water conditions to determine whether a corresponding sensed water condition requires remedial action.

27. The system of claim 26, wherein said display device is interactive to permit input of additional information that is communicated to said processor, and wherein the algorithm further employs the additional information input to said processor to determine whether at least one condition requires remedial action.

28. The system of claim 11, wherein said display device is hand-held.

29. The system of claim 11, wherein said display device is stationary.

30. The system of claim 11, wherein said display device communicates a recommendation for remediating the at least one condition detected by said sensor array that requires remedial action.

31. The system of claim 11, wherein a predetermined one of said pool cleaner and said remote display device includes a processor for analyzing the information output from said sensor array, and said display device includes means for generating an alarm signal in response to said processor determining that at least one condition detected by said sensor array requires remedial action.

32. A method of operating a pool cleaning system for cleaning at least a submerged lower wall of a pool or tank containing water, wherein the pool cleaning system comprises:
a self-propelled robotic pool cleaner that includes a cleaner housing, a sensor array including at least one sensor, the array being supported by the cleaner housing in sensing relation to the water,
a power source for supplying power to the sensor array to operate said at least one sensor, and
a remote display device,
said method comprising the steps of:
a) sensing at least one condition of the water using a sensor of the array at a first location in the pool or tank;
b) outputting information to the remote display as to each of said at least one conditions sensed by said array at said first location ;
c) displaying information relevant to the at least one condition of the water at said first location; and
d) repeating steps (a) through (c) at a second location in the pool or tank.

33. The method of claim 32, further comprising the step of generating an audible and/or visual alarm signal in response to said sensing step sensing a predetermined undesirable value for at least one of the water conditions.

34. The method of claim 32, wherein the display device includes a processor for analyzing the information from the pool cleaner, said method further comprising the step of generating an alarm signal in response to the processor determining that at least one condition sensed in said sensing step requires remedial action.

35. The method of claim 34, wherein said step of generating an alarm signal includes the step of communicating a recommended action for remediating the at least one condition that requires remedial action.

36. The method of claim 35, wherein said step of generating an alarm signal includes the step of applying an algorithm in the processor to analyze the information to determine whether at least one condition requires remedial action.

37. The method of claim 36, wherein the display device is interactive and receives the input of additional information that is communicated to the processor, and wherein said step of applying the algorithm further includes analyzing the additional information input to the processor to determine whether at least one condition requires remedial auction.

## Patentansprüche

1. Roboterpoolreinigungseinrichtung mit Selbstantrieb zum Reinigen mindestens einer unter Wasser liegenden unteren Wand eines Pools oder Behälters, der Wasser enthält, wobei die Poolreinigungseinrichtung umfasst:
ein Reinigungseinrichtungsgehäuse;
eine Sensoranordnung, die mindestens einen Sensor umfasst, wobei die Anordnung durch das Reinigungseinrichtungsgehäuse an einer Position getragen ist, an der jeder des mindestens einen Sensors in einer Erfassungsbeziehung zu dem Wasser steht, wenn sich die Poolreinigungseinrichtung bewegt, wobei jeder Sensor der Anordnung ferner einen spezifischen Zustand des Wassers erfasst, der mit der korrekten Wartung und dem korrekten Betrieb des Pools oder Behälters in Beziehung steht;
eine Stromquelle, die elektronisch mit der Sensoranordnung verbunden ist, um jeden des mindestens einen Sensors zu betreiben; und
ein Mittel zum Ausgeben einer Information über jeden durch den mindestens einen Sensor an der Anordnung erfassten Zustand an eine entfernte Anzeige, wenn sich die Poolreinigungseinrichtung durch das Wasser bewegt, wodurch der spezifische Wasserzustand an mehreren Stellen in dem Pool oder Behälter erfasst wird.

2. Poolreinigungseinrichtung nach Anspruch 1,
wobei die Sensoranordnung im Inneren des Reinigungseinrichtungsgehäuses in einem aktiven Strömungspfad des Wassers durch die Poolreinigungseinrichtung positioniert ist.

3. Poolreinigungseinrichtung nach Anspruch 1,
ferner umfassend ein Schutzgehäuse, das mindestens einen Wassereinlass umfasst und an einer Außenfläche des Reinigungseinrichtungsgehäuses angebracht ist, wobei die Sensoranordnung derart in dem Schutzgehäuse angebracht ist, dass Wasser, das durch den mindestens einen Wassereinlass in das Schutzgehäuse gelangt, in Kontakt mit dem mindestens einen Sensor in der Erfassungsbeziehung strömt.

4. Poolreinigungseinrichtung nach Anspruch 1,
wobei das Mittel zum Ausgeben einer Information ein Datenkabel umfasst, dass sich von dem Reinigungseinrichtungsgehäuse für eine Verbindung mit der entfernten Anzeige erstreckt.

5. Poolreinigungseinrichtung nach Anspruch 4,
wobei die Stromquelle ein Stromkabel umfasst, das sich von dem Reinigungseinrichtungsgehäuse für eine Verbindung mit einer entfernten elektrischen Stromquelle erstreckt, und das Datenkabel mindestens einen Leiter in dem Stromkabel umfasst.

6. Poolreinigungseinrichtung nach Anspruch 1,
wobei das Mittel zum Ausgeben einer Information einen drahtlosen Sender zum Senden der Information als drahtloses Signal von der Poolreinigungseinrichtung an die entfernte Anzeige umfasst.

7. Poolreinigungseinrichtung nach Anspruch 1,
wobei der drahtlose Sender eine Antenne mit einem freien Ende umfasst, das geeignet ist, um an einer Oberfläche des Wassers in dem Pool zu schwimmen.

8. Poolreinigungseinrichtung nach Anspruch 6,
wobei die Stormversorgung ein Stromkabel umfasst, das sich von dem Reinigungseinrichtungsgehäuse für eine Verbindung mit einer entfernten Stromquelle erstreckt.

9. Poolreinigungseinrichtung nach Anspruch 1,
wobei die Stromquelle eine Batterie in dem Reinigungseinrichtungsgehäuse umfasst.

10. Poolreinigungseinrichtung nach Anspruch 1,
ferner umfassend einen Prozessor zum Verarbeiten von Daten von der Sensoranordnung zum Erzeugen der Information, die ausgegeben werden soll.

11. Roboterpoolreinigungssystem mit Selbstantrieb zum Reinigen mindestens einer unter Wasser liegenden unteren Wand eines Pools oder Behälters, der Wasser enthält, wobei das Poolreinigungssystem umfasst:
eine Poolreinigungseinrichtung, die umfasst:
ein Reinigungseinrichtungsgehäuse, und
eine Sensoranordnung, die mindestens einen Sensor umfasst, wobei jeder des mindestens einen Sensors durch das Reinigungseinrichtungsgehäuse an einer Position in einer Erfassungsbeziehung zu dem Wasser in diesem Abschnitt des Pools oder Behälters getragen ist, wenn sich die Poolreinigungseinrichtung bewegt, wobei jeder Sensor des mindestens einen Sensors einen spezifischen Zustand des Wassers erfasst;
eine Stromquelle zum Liefern von Strom an die Sensoranordnung, um jeden des mindestens einen Sensors zu betreiben;
ein Informationsausgabemittel zum Senden einer Information über einen durch jeden des mindestens einen Sensors erfassten Zustand; und
eine entfernte Anzeigeeinrichtung in Verbindung mit dem Informationsausgabemittel, wobei der Wasserzustand an mehreren Stellen in dem Pool oder Behälter erfasst wird.

12. System nach Anspruch 11,
wobei die Sensoranordnung im Inneren des Reinigungseinrichtungsgehäuses in einem aktiven Strömungspfad des Wassers positioniert ist.

13. System nach Anspruch 11,
wobei die Poolreinigungseinrichtung ferner ein Schutzgehäuse umfasst, das mindestens einen Wassereinlass umfasst und an einer Außenfläche des Reinigungseinrichtungsgehäuses angebracht ist, wobei die Sensoranordnung derart in dem Schutzgehäuse angebracht ist, dass Wasser, das durch den mindestens einen Wassereinlass in das Schutzgehäuse gelangt, in Erfassungskontakt mit dem mindestens einen Sensor strömt.

14. System nach Anspruch 11,
wobei das Informationsausgabemittel ein Datenkabel umfasst, das sich von dem Reinigungseinrichtungsgehäuse für eine Verbindung mit der entfernten Anzeigeeinrichtung funktional erstreckt.

15. System nach Anspruch 14,
wobei die Stromquelle ein Stromkabel umfasst, das sich von dem Reinigungseinrichtungsgehäuse für eine Verbindung mit einer entfernten Stromquelle erstreckt, und das Datenkabel mindestens einen Leiter in dem Stromkabel umfasst.

16. System nach Anspruch 15,
wobei die Stromquelle in einem Stromversorgungsgehäuse positioniert ist und die entfernte Anzeigeeinrichtung in dem Stromversorgungsgehäuse umfasst ist.

17. System nach Anspruch 11,
wobei das Mittel zum Ausgeben einer Information einen drahtlosen Sender zum Übermitteln eines Signals von der Poolreinigungseinrichtung an die entfernte Anzeigeeinrichtung umfasst.

18. System nach Anspruch 17,
wobei der drahtlose Sender eine Antenne mit einem freien Ende umfasst, das geeignet ist, um an der Oberfläche des Wassers zu schwimmen.

19. System nach Anspruch 17,
wobei die Stromquelle eine wiederaufladbare Batterie umfasst, die in dem Reinigungseinrichtungsgehäuse angebracht ist.

20. System nach Anspruch 11,
wobei die Stromquelle ein Stromkabel umfasst, das sich von dem Reinigungseinrichtungsgehäuse für eine Verbindung mit einer entfernten Stromquelle erstreckt.

21. System nach Anspruch 20,
wobei die Stromquelle in einem Stromversorgungsgehäuse positioniert ist und die entfernte Anzeigeeinrichtung zu dem Stromversorgungsgehäuse gehört.

22. System nach Anspruch 11,
ferner umfassend ein Mittel zum Erzeugen eines akustischen und/oder visuell wahrnehmbaren Alarmsignals in Ansprechen darauf, dass die Sensoranordnung einen vorbestimmten nicht akzeptablen Wert für einen Wasserzustand detektiert.

23. System nach Anspruch 22,
wobei die entfernte Anzeigeeinrichtung ein Anzeigegehäuse umfasst und das Alarmerzeugungsmittel zu dem Anzeigegehäuse gehört.

24. System nach Anspruch 23,
wobei die Anzeigeeinrichtung einen akzeptablen Bereich für den Wert eines Wasserzustands und einen jüngsten Wert dieses Wasserzustands wie durch einen Sensor an der Poolreinigungseinrichtung ermittelt anzeigt.

25. System nach Anspruch 22,
wobei die Poolreinigungseinrichtung ferner einen Prozessor zum Verarbeiten von Daten von der Sensoranordnung zum Erzeugen der Information, die ausgegeben werden soll, umfasst.

26. System nach Anspruch 25,
wobei der Prozessor einen Algorithmus umfasst, der einen vorbestimmten Bereich von Werten einsetzt, der jedem eines oder mehrerer Wasserzustände entspricht, um zu ermitteln, ob ein entsprechender erfasster Wasserzustand eine Abhilfe schaffende Maßnahme erfordert.

27. System nach Anspruch 26,
wobei die Anzeigeeinrichtung interaktiv ist, um eine Eingabe einer zusätzlichen Information, die an den Prozessor übermittelt wird, zu ermöglichen, und wobei der Algorithmus ferner die zusätzliche Informationseingabe für den Prozessor einsetzt, um zu ermitteln, ob mindestens ein Zustand eine Abhilfe schaffende Maßnahme erfordert.

28. System nach Anspruch 11,
wobei die Anzeigeeinrichtung in der Hand gehalten wird.

29. System nach Anspruch 11,
wobei die Anzeigeeinrichtung stationär ist.

30. System nach Anspruch 11,
wobei die Anzeigeeinrichtung einen Vorschlag zum Korrigieren des mindestens einen durch die Sensoranordnung detektierten Zustands, der eine Abhilfe schaffende Maßnahme erfordert, übermittelt.

31. System nach Anspruch 11,
wobei eine vorbestimmte von der Poolreinigungseinrichtung und der entfernten Anzeigeeinrichtung einen Prozessor zum Analysieren der von der Sensoranordnung ausgegebenen Information umfasst, und die Anzeigeeinrichtung ein Mittel zum Erzeugen eines Alarmsignals in Ansprechen darauf, dass der Prozessor ermittelt, dass mindestens ein durch die Sensoranordnung detektierter Zustand eine Abhilfe schaffende Maßnahme erfordert, umfasst.

32. Verfahren zum Betreiben eines Poolreinigungssystems zum Reinigen mindestens einer unter Wasser liegenden unteren Wand eines Pools oder Behälters, der Wasser enthält, wobei das Poolreinigungssystem umfasst:
eine Roboterpoolreinigungseinrichtung mit Selbstantrieb, die ein Reinigungseinrichtungsgehäuse und eine Sensoranordnung umfasst, die mindestens einen Sensor umfasst, wobei die Anordnung durch das Reinigungseinrichtungsgehäuse in einer Erfassungsbeziehung zu dem Wasser getragen ist,
eine Stromquelle zum Liefern von Strom an die Sensoranordnung, um den mindestens einen Sensor zu betreiben, und eine entfernte Anzeigeeinrichtung,
wobei das Verfahren die Schritte umfasst, dass
a) mindestens ein Zustand des Wassers unter Verwendung eines Sensors der Anordnung an einer ersten Stelle in dem Pool oder Behälter erfasst wird;
b) eine Information über jeden des mindestens einen durch die Anordnung an der ersten Stelle erfassten Zustand an die entfernte Anzeige ausgegeben wird;
c) eine Information angezeigt wird, die für den mindestens einen Zustand des Wassers an der ersten Stelle relevant ist; und
d) die Schritte (a) bis (c) an einer zweiten Stelle in dem Pool oder Behälter wiederholt werden.

33. Verfahren nach Anspruch 32,
ferner umfassend den Schritt des Erzeugens eines akustischen und/oder visuell wahrnehmbaren Alarmsignals in Ansprechen darauf, dass der Erfassungsschritt einen vorbestimmten unerwünschten Wert für mindestens einen der Wasserzustände erfasst.

34. Verfahren nach Anspruch 32,
wobei die Anzeigeeinrichtung einen Prozessor zum Analysieren der Information von der Poolreinigungseinrichtung umfasst, wobei das Verfahren ferner den Schritt umfasst, dass in Ansprechen darauf, dass der Prozessor ermittelt, dass mindestens ein in dem Erfassungsschritt erfasster Zustand eine Abhilfe schaffende Maßnahme erfordert, ein Alarmsignal erzeugt wird.

35. Verfahren nach Anspruch 34,
wobei der Schritt des Erzeugens eines Alarmsignals den Schritt umfasst, dass eine vorgeschlagene Maßnahme zum Korrigieren des mindestens einen Zustands, der eine Abhilfe schaffende Maßnahme erfordert, übermittelt wird.

36. Verfahren nach Anspruch 35,
wobei der Schritt des Erzeugens eines Alarmsignals den Schritt umfasst, dass ein Algorithmus in dem Prozessor zum Analysieren der Information zum Ermitteln, ob mindestens ein Zustand eine Abhilfe schaffende Maßnahme erfordert, angewandt wird.

37. Verfahren nach Anspruch 36,
wobei die Anzeigeeinrichtung interaktiv ist und die Eingabe einer zusätzlichen Information, die an den Prozessor übermittelt wird, empfängt, und wobei der Schritt des Anwendens des Algorithmus ferner umfasst, dass die zusätzliche Informationseingabe für den Prozessor analysiert wird, um zu ermitteln, ob mindestens ein Zustand eine Abhilfe schaffende Maßnahme erfordert.

## Revendications

1. Système de nettoyage de piscine robotisé autopropulsé pour nettoyer au moins une paroi inférieure immergée d'une piscine ou d'un réservoir contenant de l'eau, ledit système de nettoyage de piscine comportant :
un boîtier du système de nettoyage,
une rangée de détecteurs incluant au moins un détecteur, ladite rangée étant supportée par ledit boîtier du système de nettoyage dans une position au niveau de laquelle chacun dudit au moins un détecteur est dans une relation de détection par rapport à l'eau lorsque ledit système de nettoyage de piscine est en mouvement, chaque détecteur de ladite rangée détectant en outre une condition spécifique de l'eau, laquelle est associée à la maintenance et au fonctionnement appropriés de la piscine ou du réservoir,
une source d'alimentation connectée électroniquement à ladite rangée de détecteurs pour mettre en fonctionnement chacun dudit au moins un détecteur, et
des moyens pour délivrer en sortie des informations à un écran distant concernant chaque condition détectée par ledit au moins un détecteur sur ladite rangée lorsque ledit système de nettoyage de piscine se déplace dans l'eau, la condition d'eau spécifique étant détectée à une pluralité d'emplacements dans la piscine ou le réservoir.

2. Système de nettoyage de piscine selon la revendication 1, dans lequel ladite rangée de détecteurs est positionnée à l'intérieur dudit boîtier du système de nettoyage dans un trajet de débit actif d'eau à travers ledit système de nettoyage de piscine.

3. Système de nettoyage de piscine selon la revendication 1, comportant en outre un boîtier de protection incluant au moins une alimentation en eau montée sur une surface extérieure dudit boîtier du système de nettoyage, ladite rangée de détecteurs étant montée dans ledit boîtier de protection de sorte que de l'eau pénétrant dans ledit boîtier de protection à travers ladite au moins une alimentation en eau circule en contact avec ledit au moins un détecteur dans ladite relation de détection.

4. Système de nettoyage de piscine selon la revendication 1, dans lequel lesdits moyens pour délivrer en sortie des informations incluent un câble de données s'étendant depuis le boîtier du système de nettoyage en vue d'une connexion à l'écran distant.

5. Système de nettoyage de piscine selon la revendication 4, dans lequel ladite source d'alimentation inclut un câble d'alimentation s'étendant depuis ledit boîtier du système de nettoyage en vue d'une connexion à une source distante de courant électrique, et ledit câble de données comporte au moins un conducteur dans ledit câble d'alimentation.

6. Système de nettoyage de piscine selon la revendication 1, dans lequel lesdits moyens pour délivrer en sortie des informations incluent un émetteur sans fil pour transmettre les informations sous forme d'un signal sans fil depuis ledit système de nettoyage de piscine à l'écran distant.

7. Système de nettoyage de piscine selon la revendication 1, dans lequel ledit émetteur sans fil inclut une antenne ayant une extrémité libre adaptée pour flotter sur une surface supérieure de l'eau dans la piscine.

8. Système de nettoyage de piscine selon la revendication 6, dans lequel ladite source d'alimentation inclut un câble d'alimentation s'étendant depuis ledit boîtier du système de nettoyage en vue d'une connexion à une source d'alimentation distante.

9. Système de nettoyage de piscine selon la revendication 1, dans lequel ladite source d'alimentation inclut une batterie dans ledit boîtier du système de nettoyage.

10. Système de nettoyage de piscine selon la revendication 1, comportant en outre un processeur pour traiter des données depuis ladite rangée de détecteurs afin de générer les informations à délivrer en sortie.

11. Système de nettoyage de piscine robotisé autopropulsé pour nettoyer au moins une paroi inférieure immergée d'une piscine ou d'un réservoir contenant de l'eau, ledit système de nettoyage de piscine, comportant :
un système de nettoyage de piscine qui inclut
un boîtier du système de nettoyage, et
une rangée de détecteurs incluant au moins un détecteur, chacun dudit au moins un détecteur étant supporté par ledit boîtier du système de nettoyage dans une position en relation de détection par rapport à l'eau dans cette partie de la piscine ou du réservoir lorsque ledit système de nettoyage de piscine est en mouvement, chaque détecteur dudit au moins un détecteur détectant une condition spécifique de l'eau,
une source d'alimentation pour délivrer de l'énergie à ladite rangée de détecteurs pour mettre en fonctionnement chacun dudit au moins un détecteur,
des moyens de sortie d'informations pour transmettre des informations sur une condition détectée par chacun dudit au moins un détecteur, et
un dispositif d'affichage distant en communication avec lesdits moyens de sortie d'informations,
la condition d'eau étant détectée au niveau d'une pluralité d'emplacements dans la piscine ou le réservoir.

12. Système selon la revendication 11, dans lequel ladite rangée de détecteurs est positionnée à l'intérieur dudit boîtier du système de nettoyage dans un trajet de débit actif d'eau.

13. Système selon la revendication 11, ledit système de nettoyage de piscine comportant en outre un boîtier de protection incluant au moins une alimentation en eau montée sur une surface extérieure dudit boîtier du système de nettoyage, ladite rangée de détecteurs étant montée dans ledit boîtier de protection de sorte que l'eau pénétrant dans ledit boîtier de protection à travers ladite au moins une alimentation en eau circule en contact de détection avec ledit au moins un détecteur.

14. Système selon la revendication 11, dans lequel lesdits moyens de sortie d'informations incluent un câble de données s'étendant de manière opérationnelle depuis ledit boîtier du système de nettoyage en vue d'une connexion audit dispositif d'affichage distant.

15. Système selon la revendication 14, dans lequel ladite source d'alimentation inclut un câble d'alimentation s'étendant depuis ledit boîtier du système de nettoyage en vue d'une connexion à une source d'alimentation distante, et ledit câble de données comporte au moins un conducteur dans ledit câble d'alimentation.

16. Système selon la revendication 15, dans lequel la source d'alimentation est positionnée dans un boîtier de source d'alimentation et ledit dispositif d'affichage distant est inclus dans le boîtier de source d'alimentation.

17. Système selon la revendication 11, dans lequel lesdits moyens pour délivrer en sortie des informations incluent un émetteur sans fil pour communiquer un signal depuis ledit système de nettoyage de piscine audit dispositif d'affichage distant.

18. Système selon la revendication 17, dans lequel ledit émetteur sans fil inclut une antenne ayant une extrémité libre adaptée pour flotter à la surface de l'eau.

19. Système selon la revendication 17, dans lequel ladite source d'alimentation inclut une batterie rechargeable montée dans ledit boîtier du système de nettoyage.

20. Système selon la revendication 11, dans lequel ladite source d'alimentation inclut un câble d'alimentation s'étendant depuis ledit boîtier du système de nettoyage en vue d'une connexion à une source d'alimentation distante.

21. Système selon la revendication 20, dans lequel la source d'alimentation est positionnée dans un boîtier de source d'alimentation et ledit dispositif d'affichage distant est associé au boîtier de source d'alimentation.

22. Système selon la revendication 11, comportant en outre des moyens pour générer un signal d'alarme audible et/ou visible en réponse à ladite rangée de détecteurs détectant une valeur inacceptable prédéterminée pour une condition d'eau.

23. Système selon la revendication 22, dans lequel ledit dispositif d'affichage distant inclut un boîtier d'affichage et lesdits moyens de génération d'alarme sont associés audit boîtier d'affichage.

24. Système selon la revendication 23, dans lequel ledit dispositif d'affichage affiche une plage acceptable pour la valeur d'une condition d'eau et une valeur la plus récente de la même condition d'eau comme déterminé par un détecteur sur le système de nettoyage de piscine.

25. Système selon la revendication 22, dans lequel ledit système de nettoyage de piscine comporte en outre un processeur pour traiter des données depuis ladite rangée de détecteurs afin de générer les informations à délivrer en sortie.

26. Système selon la revendication 25, dans lequel ledit processeur inclut un algorithme utilisant une plage prédéterminée de valeurs correspondant à chacune parmi une ou plusieurs conditions d'eau afin de déterminer si une condition d'eau détectée correspondante nécessite des mesures correctives.

27. Système selon la revendication 26, dans lequel ledit dispositif d'affichage est interactif pour permettre l'entrée d'informations supplémentaires qui sont communiquées audit processeur, et dans lequel l'algorithme utilise en outre les informations supplémentaires entrées dans ledit processeur pour déterminer si au moins une condition nécessite des mesures correctives.

28. Système selon la revendication 11, dans lequel ledit dispositif d'affichage est portable.

29. Système selon la revendication 11, dans lequel ledit dispositif d'affichage est stationnaire.

30. Système selon la revendication 11, dans lequel ledit dispositif d'affichage communique une recommandation pour remédier à la au moins une condition détectée par ladite rangée de détecteurs qui nécessite des mesures correctives.

31. Système selon la revendication 11, dans lequel l'un prédéterminé parmi ledit système de nettoyage de piscine et ledit dispositif d'affichage distant inclut un processeur pour analyser les informations délivrées en sortie par ladite rangée de détecteurs, et ledit dispositif d'affichage inclut des moyens pour générer un signal d'alarme en réponse audit processeur déterminant qu'au moins une condition détectée par ladite rangée de détecteurs nécessite des mesures correctives.

32. Procédé de fonctionnement d'un système de nettoyage de piscine pour nettoyer au moins une paroi inférieure immergée d'une piscine ou d'un réservoir contenant de l'eau, le système de nettoyage de piscine comportant :
un système de nettoyage de piscine robotisé autopropulsé qui inclut un boîtier du système de nettoyage, une rangée de détecteurs incluant au moins un détecteur, la rangée étant supportée par le boîtier du système de nettoyage dans une relation de détection par rapport à l'eau,
une source d'alimentation pour délivrer de l'énergie à la rangée de détecteurs afin de faire fonctionner ledit au moins un détecteur, et
un dispositif d'affichage distant,
ledit procédé comportant les étapes consistant à :
a) détecter au moins une condition de l'eau en utilisant un détecteur de la rangée au niveau d'un premier emplacement dans la piscine ou le réservoir,
b) délivrer en sortie des informations au dispositif distant concernant chacune desdites au moins une condition détectée par ladite rangée au niveau dudit premier emplacement,
c) afficher des informations appropriées à la au moins une condition de l'eau au niveau dudit premier emplacement, et
d) répéter les étapes a) à c) au niveau d'un second emplacement dans la piscine ou le réservoir.

33. Procédé selon la revendication 32, comportant en outre l'étape consistant à générer un signal d'alarme audible et/ou visuel en réponse à ladite étape de détection détectant une valeur indésirable prédéterminée pour au moins l'une des conditions d'eau.

34. Procédé selon la revendication 32, dans lequel le dispositif d'affichage inclut un processeur pour analyser les informations provenant du système de nettoyage de piscine, ledit procédé comportant en outre l'étape consistant à générer un signal d'alarme en réponse au processeur déterminant qu'au moins une condition détectée à ladite étape de détection nécessite des mesures correctives.

35. Procédé selon la revendication 34, dans lequel ladite étape de génération d'un signal d'alarme inclut l'étape consistant à communiquer une action recommandée pour remédier à la au moins une condition qui nécessite des mesures correctives.

36. Procédé selon la revendication 35, dans lequel ladite étape de génération d'un signal d'alarme inclut l'étape consistant à appliquer un algorithme dans le processeur pour analyser les informations afin de déterminer si au moins une condition nécessite des mesures correctives.

37. Procédé selon la revendication 36, dans lequel le dispositif d'affichage est interactif et reçoit l'entrée d'informations supplémentaires qui sont communiquées au processeur, et dans lequel ladite étape d'application de l'algorithme inclut en outre l'analyse d'informations supplémentaires entrées dans le processeur pour déterminer si au moins une condition nécessite des mesures correctives.
